(19) Europäisches Patentamt
European Patent Office
Office européen des brevets

(11) EP 0 821 932 A1

# (12) EUROPÄISCHE PATENTANMELDUNG

(43) Veröffentlichungstag:
**04.02.1998 Patentblatt 1998/06**

(51) Int. Cl.$^6$: **A61K 7/06**, C08G 69/10, C08G 73/06

(21) Anmeldenummer: **97113350.9**

(22) Anmeldetag: **01.08.1997**

(84) Benannte Vertragsstaaten:
**AT BE CH DE DK ES FI FR GB GR IE IT LI LU MC NL PT SE**

(30) Priorität: **02.08.1996 DE 19631379**

(71) Anmelder:
**BASF AKTIENGESELLSCHAFT**
**67056 Ludwigshafen (DE)**

(72) Erfinder:
- **Nguyen Kim, Son, Dr.**
  **69502 Hemsbach (DE)**
- **Sanner, Axel, Dr.**
  **67227 Frankenthal (DE)**
- **Hössel, Peter, Dr.**
  **67105 Schifferstadt (DE)**
- **Kroner, Matthias, Dr.**
  **67304 Eisenberg (DE)**

(74) Vertreter:
**Kinzebach, Werner, Dr. et al**
**Patentanwälte**
**Reitstötter, Kinzebach und Partner**
**Postfach 86 06 49**
**81633 München (DE)**

## (54) Wasserlösliche oder wasserdispergierbare Polyasparaginsäure-Derivate, ihre Herstellung und ihre Verwendung

(57) Die vorliegende Erfindung betrifft wasserlösliche oder wasserdispergierbare Polyasparaginsäurederivate, ihre Herstellung und ihre Verwendung in der Kosmetik, insbesondere in der Haarkosmetik.

EP 0 821 932 A1

## Beschreibung

Die vorliegende Erfindung betrifft wasserlösliche oder wasserdispergierbare Polyasparaginsäure-Derivate, ihre Herstellung und ihre Verwendung in der Kosmetik.

Synthetisch hergestellte Polyaminosäuren und ihre Derivate sind seit langem bekannt und werden aufgrund ihrer biologischen Verträglichkeit, z.B. für spezielle Anwendungen in der Medizin und Pharmazie verwendet. Abgesehen von Peptiden mit wirkungsspezifischer Sequenz, handelt es sich dabei vor allem um filmbildende Substanzen zur Verbesserung der Handhabbarkeit von pharmazeutischen Zubereitungen, zur Verbesserung ihrer Lagerstabilität und vor allem zur Beeinflussung der Abgabegeschwindigkeit der Wirksubstanzen. Dabei werden z.B. Polymere aus einzelnen Aminosäuren, wie Polyasparaginsäure, Polyglutaminsäure und Polylysin sowie Mischpolymerisate und biologisch gut verträgliche Derivate solcher Polyaminosäuren verwendet.

Die DE-A-37 00 128 beschreibt Poly-(hydroxyalkyl)-aminodicarbonsäurederivate mit biologisch inaktiven Acylgruppen, Verfahren zu ihrer Herstellung und ihre Verwendung für Wirkstoffdepotzubereitungen mit kontrollierter Wirkstoffabgabe.

Die DE-A-36 12 102 beschreibt lösliche und biologisch abbaubare Mischpolymerisate aus Asparaginsäure- und/oder Glutaminsäure-Einheiten, die reaktive Gruppen, wie z.B. Hydrazid oder Azidgruppen, für die chemische Anbindung von biologisch aktiven Stoffen enthalten.

Die EP-B-0 406 623 beschreibt filmbildende Polyasparaginsäure-Derivate, die durch Umsetzung von Polysuccinimid mit Aminen erhalten werden und Struktureinheiten entsprechend der folgenden Formel

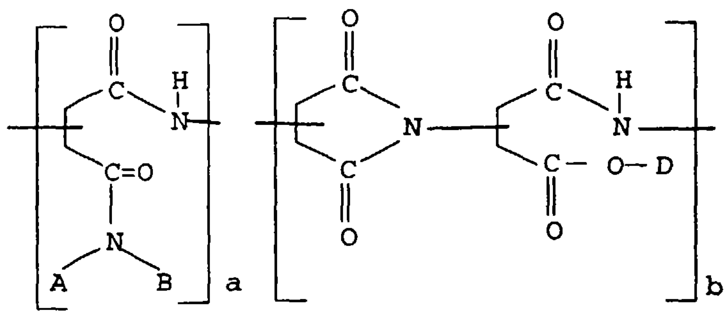

enthalten, in der

A = H oder ein Alkylrest bzw. ein Alkylenrest mit 1 bis 8 C-Atomen ist, der auch verzweigt und noch mit cycloaliphatischen oder aromatischen Resten, wobei der ringförmige Substituent auch Heteroatome enthalten, oder mit R-O-Gruppen, wobei R = H oder gegebenenfalls eine verzweigte Alkylgruppe oder Cycloalkylgruppe mit 1 bis 10 C-Atomen sein kann, substituiert sein kann,

B = H oder ein wie unter A definierter Alkyl- bzw. Alkylenrest ist, der gleich oder verschieden von A sein kann,

D = H oder $NH_4$ oder

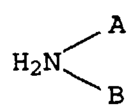

mit den obenangegebenen Bedeutungen von A und B oder ein Alkali und

a = 0,2 bis 1 und

b = 0,8 bis 0

sind, die als Überzugsmittel und/oder Retardierungsmittel für Arzneiformen von therapeutischen Wirkstoffen und für Lebensmittel und Genußmittel verwendet werden. Wie die obige Formel zeigt, sind dabei die Monomereinheiten, die in Salzform vorliegen immer an eine Imidmonomereinheit gebunden. Das stöchiometrische Verhältnis von Monomereinheiten in Salzform zu Imidmonomereinheiten ist auf 1:1 festgelegt.

Auch eine kosmetische Verwendung von Polyaminosäuren und ihren Derivaten ist bereits beschrieben worden. Polypeptide aus Proteinhydrolysaten, z.B. auf Eiweiß- oder Kollagenbasis, sind im Handel erhältlich. So werden z.B. Proteinhydrolysate oder Partialhydrolysate aus Kollagen mit eine Mol-Gew. von 1100 bis 1300 und etwa 8 bis 14 Monomer-Einheiten und deren Natriumsalze (z.B. Nutrilan® der Fa. Grünau) als Schutzkolloide, die nicht schäumen und nicht waschaktiv sind, aber dispergierende und schmutztragende Eigenschaften aufweisen, z.B. in Kombination mit Tensiden verwendet. Ebenso sind verschiedene Fettsäure-Polypeptid-Kondensationsprodukte als biologisch abbaubare anionische Tenside mit guten Schaum- und Waschvermögen im Handel erhältlich (z.B. Lamepon® der Fa. Grünau).

Die DE-A-22 53 190 beschreibt Polyasparaginsäurederivate mit Säureamid- und Alkali- bzw. Erdalkalicarboxylat-Resten, ihre Herstellung durch Umsetzung von Polysuccinimid mit einem Molekulargewicht von 300 bis 30000 mit einem primären oder sekundären Amin und anschliessender Hydrolyse mit einem Alkali- oder Erdalkalihydroxid oder -carbonat sowie die Verwendung der erhaltenen Produkte als Tenside und Zusatzstoffe für Waschmittel und Kosmetika.

Die JP-A-0624 8072 beschreibt die kosmetische Verwendung von Polyasparaginsäureamiden mit Alkalicarboxylatresten.

Im Bereich der Kosmetik besteht ein grosser Bedarf an in Wasser löslichen oder dispergierbaren Polymeren, die gute biologische Verträglichkeit und biologische Abbaubarkeit sowie filmbildende Eigenschaften aufweisen. Solche Filmbildnerpolymere werden z.B. zur Festigung, Strukturverbesserung und Formgebung der Haare verwendet. Die Haarbehandlungsmittel enthalten im allgemeinen eine Lösung des Filmbildners in einem Alkohol oder einem Gemisch aus Alkohol und Wasser und werden z.B. in Form dieser wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht (K-Wert > 14 nach E. Fikentscher, Cellulosechemie 13 (1932), S. 58-64) besitzen. Diese Polymere sind aber aufgrund des höheren Molekulargewichtes im allgemeinen schlechter auswaschbar.

Eine Verwendung der obenbeschriebenen Polymere auf Basis von Polyaminosäurederivaten in der Kosmetik zur Festigung, Strukturverbesserung und Formgebung der Haare ist bisher noch nicht beschrieben. Außerdem erfüllen die bekannten Polymere nicht die Anforderung, bei guten Festigereigeschaften dennoch leicht auswaschbar zu sein.

Der vorliegenden Erfindung liegt daher die Aufgabe zugrunde, Haarbehandlungsmittel zur Verfügung zu stellen, die einerseits als Haarfestiger brauchbar sind, andererseits aber auch verbesserte Auswaschbarkeit (Redispergierbarkeit) besitzen.

Überraschenderweise wurde nun gefunden, daß diese Aufgabe durch wasserlösliche oder wasserdispergierbare Polyasparaginsäurederivate gelöst wird, welche Umsetzungsprodukte aus Polysuccinimid oder Polyasparaginsäure mit einem Amingemisch, das mindestens ein primäres oder sekundäres Amin enthält, sind.

Gegenstand der vorliegenden Erfindung ist daher die Verwendung von wasserlöslichen und/oder wasserdispergierbaren Polyasparaginsäurederivaten auf Basis der in der schematisierten Formel I gezeigten Einheiten

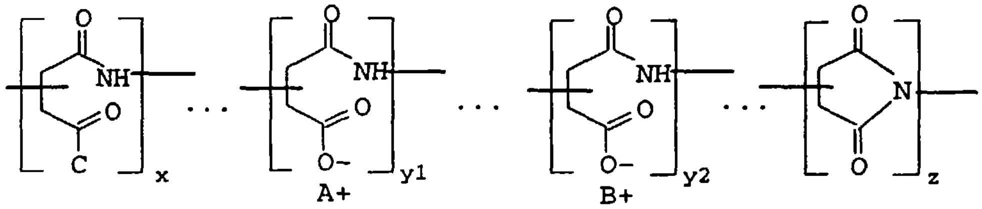

(I)

wobei
die Reihenfolge der Einheiten beliebig ist,
die Summe aus x + y1 + y2 + z = 100 ist und

- x für einen Wert von 30 bis 100 steht,
- y1 für einen Wert von 0 bis 70 steht,
- y2 für einen Wert von 0 bis 50 steht,
- z für einen Wert von 0 bis 30 steht,
- A für mindestens ein primäres oder sekundäres nichtaromatisches Amin steht,
- B für mindestens ein Amin mit einer tertiären Aminogruppe steht,
- C für einen von einem Amin des Typs A durch Abspaltung eines Aminwasserstoffs abgeleiteten Rest steht,

oder deren Carbonsäure- und Polycarbonsäuresalze oder Quaternisierungsprodukte,
in der Kosmetik, insbesondere in der Haarkosmetik.

Im Rahmen der vorliegenden Erfindung steht der Ausdruck "Alkyl" für geradkettige und verzweigte Alkylgruppen. Vorzugsweise handelt es sich dabei um geradkettige oder verzweigte $C_1$-$C_{30}$-Alkylgruppen, bevorzugt $C_3$-$C_{10}$-Alkyl-, insbesondere $C_4$-$C_8$-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, n-Hexyl, Ethylhexyl, n-Heptyl und Octyl.

Die obigen Ausführungen zur Alkylgruppe gelten in entsprechender Weise für die Alkylgruppe in Alkoxy, Alkoxycarbonyl und Alkylaminocarbonyl.

Bei der Cycloalkylgruppe handelt es sich vorzugsweise um eine $C_5$-$C_7$-Cycloalkylgruppe, wie Cyclopentyl, Cyclohexyl oder Cycloheptyl. Wenn die Cycloalkylgruppe substituiert ist, weist sie vorzugsweise 1 oder 2 Substituenten auf.

Cycloalkenyl steht vorzugsweise für eine $C_5$-$C_7$-Cycloalkenylgruppe, wie Cyclopentenyl, Cyclohexenyl oder Cycloheptenyl. Wenn die Cycloalkenylgruppe substituiert ist, weist sie vorzugsweise 1 oder 2 Substituenten auf.

Bicycloalkyl steht vorzugsweise für Decalinyl, Indanyl, Hydrindanyl, Bornyl, Pinanyl, Caranyl, Norbornyl und Bicyclo[2.2.2]octanyl.

Bicycloalkenyl kann eine oder zwei Doppelbindungen aufweisen und steht vorzugsweise für Indenyl, Pinenyl, Norbornenyl und Norbornadienyl.

Aryl steht vorzugsweise für Phenyl.

Hetaryl steht vorzugsweise für einen 5- oder 6-gliedrigen aromatischen Heterocyclus, der 1 oder 2 Heteroatome aufweist, die unabhängig voneinander ausgewählt sind unter N, O und S. Insbesondere handelt es sich dabei um Pyridinyl, Pyrimidinyl, Pyrazinyl, Thienyl, Oxazolyl, Imidazolyl, Pyrrolyl und Furanyl.

Heterocyclyl steht vorzugsweise für einen 5- bis 7-gliedrigen, gesättigten oder ungesättigten Heterocyclus, der 1, 2 oder 3 Heteroatome aufweist, die unabhängig voneinander ausgewählt sind unter N, O und S. Handelt es sich bei dein Heteroatom um Stickstoff, so kann dieser zusätzlich gegebenenfalls alkyliert oder acyliert sein. Beispiele sind Pyrrolidinyl, Imidazolidinyl, Piperidinyl und Morpholinyl.

Wenn die Aryl-, Hetaryl- oder Heterocyclylgruppen substituiert sind, so weisen sie vorzugsweise 1 oder 2 Substituenten in beliebiger Position auf.

Der Rest C leitet sich von einem Amin des Typs A ab, bei dem ein Aminwasserstoff in einer Kondensationsreaktion unter Ausbildung einer Amidbindung abgespalten wurde.

Der Rest $A^+$ leitet sich von einem Amin des Typs A ab, das mit einer Carboxylgruppe ein Ammoniumsalz gebildet hat.

Der Rest $B^+$ leitet sich von einem Amin des Typs B ab, das mit einer Carboxylgruppe ein Ammoniumsalz gebildet hat.

Bei den Endgruppen der Polymere handelt es sich um Reste, die zwei freie terminale Carboxylgruppen oder eine freie terminale Aminogruppe und eine freie Carboxylgruppe aufweisen sowie gegebenenfalls um deren partielle oder vollständige Quaternisierungsprodukte oder gegebenenfalls deren Salze mit Aminen vom Typ A oder B oder gegebenenfalls deren Salze mit zur Neutralisierung verwendeter Carbon- oder Polycarbonsäuren.

Bevorzugt sind die Amine A ausgewählt unter

1) primären Aminen der Formel II

$$R^1\text{-}NH_2 \qquad \text{(II)}$$

worin $R^1$ für

a) Alkyl steht, welches mit 1, 2 oder 3 Gruppen substituiert sein kann, die unabhängig voneinander ausgewählt sind unter Amino, Hydroxy, Alkoxycarbonyl, Alkylaminocarbonyl, Cycloalkyl, Cycloalkenyl, Aryl oder einem 5- bis 7-gliedrigen gesättigten, ungesättigten oder aromatischen Heterocyclus, der 1, 2 oder 3 N-, S- und/oder O-Heteroatome aufweist, wobei die cyclischen Reste zusätzlich 1, 2, oder 3 Substituenten, unabhängig ausgewählt unter Alkyl, Alkoxy, Hydroxy und Hydroxyalkyl, aufweisen können und/oder die Alkylgruppe durch 1, 2 oder 3 nicht benachbarte Sauerstoffatome unterbrochen sein kann;

b) Cycloalkyl oder Cycloalkenyl steht, wobei diese Reste mit 1, 2 oder 3 der folgenden Gruppen substituiert sein können, die unabhängig voneinander ausgewählt sind unter Alkyl, Alkoxy, Hydroxy, Hydroxyalkyl oder -$COR^2$, worin $R^2$ für Alkyl, Alkoxy oder $NR^3R^4$ steht, wobei $R^3$ und $R^4$ unabhängig voneinander für Wasserstoff und Alkyl stehen;

c) Bicycloalkyl oder Bicycloalkenyl steht, wobei diese Reste mit 1, 2 oder 3 der unter b) als Substituenten für Cycloalkyl und Cycloalkenyl angegebenen Gruppen substituiert sein können;

d) einen 5- bis 7-gliedrigen, gesättigten Heterocyclus steht, der 1, 2 oder 3 Heteroatome, ausgewählt unter O,

N und S aufweist und der gegebenenfalls 1, 2 oder 3 Substituenten aufweisen kann, die unabhängig voneinander ausgewählt sind unter Alkyl, Alkoxy, Hydroxy und Hydroxyalkyl;

2) sekundären Aminen der Formel III

$$R^5 - NH - R^6 \qquad (III)$$

worin

$R^5$ und $R^6$ unabhängig voneinander die für $R^1$ angegebenen Bedeutungen annehmen können, oder

$R^5$ und $R^6$ zusammen mit dem Stickstoffatom, an das sie gebunden sind für einen 5- bis 7-gliedrigen, gesättigten oder ungesättigten Heterocyclus stehen, der 1, 2 oder 3 Heteroatome oder -gruppen, unabhängig voneinander ausgewählt unter $NR^7$, $NCOR^7$, O und S aufweisen kann, wobei $R^7$ für Wasserstoff oder Alkyl steht, und/oder wobei der Heterocyclus durch 1, 2 oder 3 Substituenten, unabhängig voneinander ausgewählt unter Alkyl, Alkoxy, Hydroxy und Hydroxyalkyl, substituiert sein kann.

Vorzugsweise handelt es sich bei mindestens einem der Amine A um ein primäres Amin der Formel II, worin $R^1$ für Alkyl oder Cycloalkyl steht.

A steht insbesondere für Cyclohexylamin.

Gemäß einer weiteren bevorzugten Ausführungsform handelt es sich bei mindestens einem der Amine A um ein primäres Amin der Formel II, worin $R^1$ für Amino steht.

A steht dann insbesondere für Ethylendiamin.

Gemäß einer weiteren bevorzugten Ausführungsform handelt es sich bei mindestens einem der Amine A um ein primäres Amin der Formel II, worin $R^1$ für Hydroxyalkyl steht.

A steht dann beispielsweise für Pentanolamin, Hexanolamin, Heptanolamin oder Octanolamin.

Gemäß einer weiteren bevorzugten Ausführungsform handelt es sich bei mindestens einem der Amine A um ein primäres Amin der Formel II, worin $R^1$ für Cycloalkylalkyl oder Arylalkyl steht.

A steht dann beispielsweise für Cyclopentylmethylamin, Cyclohexylmethylamin oder Benzylamin.

Vorzugsweise handelt es sich bei mindestens einem der Amine A um ein primäres Amin der Formel II, worin $R^1$ für einen Alkoxycarbonylalkylrest steht.

A steht dann beispielsweise für Ethyl-, Propyl-, Butyl-, tert.-Butylalaninat, Ethyl-, Propyl-, Butyl-, tert.-Butylvalinat, Ethyl-, Propyl-, Butyl-, tert.-Butylleucinat, Ethyl-, Propyl-, Butylglycinat, und insbesondere für tert.-Butylglycinat.

Gemäß einer weiteren bevorzugten Ausführungsform handelt es sich bei mindestens einem der Amine A um ein primäres Amin der Formel II, worin $R^1$ für einen Alkylaminocarbonylalkylrest steht.

A steht dann beispielsweise für die Ethyl-, Propyl-, Butyl- oder tert.-Butyl-Carbonsäureamide der Aminosäuren Glycin, Alanin, Valin, Leucin oder Isoleucin.

Vorzugsweise handelt es sich bei mindestens einem der Amine A um ein sekundäres Amin der Formel III, worin $R^5$ und $R^6$ unabhängig voneinander für Alkyl und Cycloalkyl stehen.

Gemäß einer weiteren bevorzugten Ausführungsform handelt es sich bei mindestens einem der Amine A um ein sekundäres Amin der Formel III, worin $R^5$ für Alkyl, Cycloalkyl oder Hydroxyalkyl und $R^6$ für Hydroxyalkyl steht.

A steht dann beispielsweise für N-Methylethanolamin, N-Methylisopropanolamin, N-Ethylethanolamin, N-Ethylisopropanolamin, 2,2'-Iminodiethanol und 2,2'-Iminodipropanol.

Gemäß einer weiteren bevorzugten Ausführungsform handelt es sich bei mindestens einem der Amine A um ein sekundäres Amin der Formel III, worin $R^5$ und $R^6$ zusammen mit dem Stickstoffatom, an das sie gebunden sind, für einen 5- bis 7-gliedrigen, gesättigten Heterocyclus stehen, der ein weiteres Heteroatom, ausgewählt unter S, O, $NR^7$ oder $NCOR^7$ aufweisen kann, wobei $R^7$ für H, Alkyl oder Hydroxyalkyl steht, und der zusätzlich einen oder zwei Substituenten, ausgewählt unter Alkyl oder Hydroxy, aufweisen kann.

Vorzugsweise steht A dann für Pyrrolidin, Imidazolidin, Piperidin, 4-Methylpiperidin, 3-Piperidinol, 4-Piperidinol, Morpholin, 2,6-Dimethylmorpholin, Piperazin, 1-Methylpiperazin, 1-Ethylpiperazin, N-(2-Hydroxyethyl)piperazin, N-Acylpiperazin und insbesondere für Piperidin, Morpholin und 1-Methylpiperazin.

In einer bevorzugten Ausführung werden Polyasparaginsäurederivate verwendet, für die die Summe der Indizes x+y1 in der schematisierten Formel I für einen Wert von 50 oder größer, bevorzugt 75 oder größer steht, d.h. die eine dementsprechende Anzahl an von Aminen des Typs A abgeleiteten Resten aufweisen. Weiterhin bevorzugt ist die Verwendung von Derivaten, für die der Index y2 in der schematisierten Formel I für einen Wert von 5 oder größer steht, d.h. die eine dementsprechende Anzahl an von Aminen des Typs B abgeleiteten Resten aufweisen.

Geeignete Amine vom Typ B sind

1) tertiäre Amine der Formel IV

$NR^8R^9R^{10}$ (IV)

worin

$R^8$, $R^9$ und $R^{10}$ unabhängig voneinander die für $R^1$ angegebenen Bedeutungen annehmen können, oder
$R^8$ und $R^9$ zusammen mit dem Stickstoffatom, an das sie gebunden sind, für einen 5- bis 7-gliedrigen gesättigten oder ungesättigten Heterocyclus stehen, der 1, 2 oder 3 Heteroatome, ausgewählt unter $NR^{11}$, $NCOR^{11}$ und O, wobei $R^{11}$ für Wasserstoff oder Alkyl steht, aufweisen kann, und/oder der durch 1, 2 oder 3 Substituenten, unabhängig voneinander ausgewählt unter Alkyl, Alkoxy, Hydroxy und Hydroxyalkyl, substituiert sein kann;

2) Diamine der Formel V

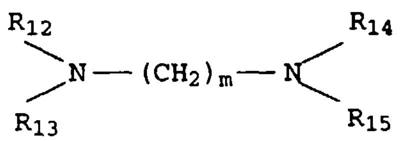

(V)

wobei

m für eine ganze Zahl von 2 bis 6 steht,
$R^{12}$ und $R^{13}$ unabhängig voneinander für Wasserstoff und Alkyl stehen,
$R^{14}$ und $R^{15}$ unabhängig voneinander für Alkyl stehen oder $R^{14}$ und $R^{15}$ zusammen mit dem Stickstoffatom, an das sie gebunden sind, für einen 5- bis 7-gliedrigen gesättigten Heterocyclus stehen, der 1, 2 oder 3 Heteroatome, ausgewählt unter N, O und S aufweist;

3) tertiäre Fettamine der Formel VI

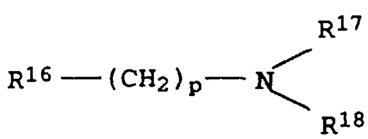

(VI)

worin

p für eine ganze Zahl von 6 bis 23 steht,
$R^{16}$ für Wasserstoff oder den Kohlenwasserstoffrest einer gesättigten oder ungesättigten Fettsäure steht,
$R^{17}$ und $R^{18}$ unabhängig voneinander für Alkyl stehen.

Gemäß einer bevorzugten Ausführungsform handelt es sich bei mindestens einem der Amine B um ein Amin der Formel IV, worin $R^8$ und $R^9$ zusammen mit dem Stickstoffatom, an das sie gebunden sind, für einen 5- bis 7-gliedrigen, gesättigten Heterocyclus stehen, der ein weiteres Heteroatom, ausgewählt unter S, O, $NR^7$ oder $NCOR^7$ aufweisen kann, wobei $R^7$ für H, Alkyl oder Hydroxyalkyl steht, und der zusätzlich einen oder zwei Substiuenten, ausgewählt unter Alkyl oder Hydroxy, aufweisen kann.

Vorzugsweise steht B dann für N-Methylpyrrolidin, N-Methylpiperidin, N-Methylpiperazin, N-Ethylpiperazin, N,N'-Dimethylpiperazin und insbesondere für N-Methylpiperazin.

Vorzugsweise beträgt der Molanteil an quaternären Ammoniumgruppen in den Polyasparaginsäurederivaten nach der Formel I, ausgedrückt als Summe von y1+y2 50 % und mehr, insbesondere 70 % und mehr. Diese Derivate weisen eine gute biologische Abbaubarkeit auf.

Gemäß einer speziellen Ausführungsform verwendet man jedoch auch Polyasparaginsäurederivate mit Monomereinheiten nach der Formel I, wobei die Summe y1+y2 = 0 ist, d.h. die keine quaternären Ammoniumgruppen enthalten.

Zur Herstellung von Polyasparaginsäurederivaten mit Monomereinheiten der Formel I, wobei y1+y2 = 0 ist, wird das Polysuccinimid oder die Polyasparaginsäure in einem geeigneten polaren Lösungsmittel, z.B. N-Methlypyrrolidon, gelöst. Zweckmäßigerweise erfolgt das Lösen bei erhöhter Temperatur, z.B. 100 bis 130°C. Zu der Lösung gibt man

dann eine Aminmischung mit mindestens einem Amin des vorbeschriebenen Typs A. Die Reaktionstemperatur liegt im allgemeinen im Bereich von 0°C bis 80°C und die Reaktionszeit beträgt 1 Stunde bis zu mehreren Tagen. Das Produkt wird dann in üblicher Weise gewonnen, z.B. durch Ausfällen mit einem organischen Lösungsmittel, wie Aceton.

Ein weiterer Gegenstand der Erfindung sind auch wasserlösliche und/oder wasserdispergierbare Polyasparaginsäurederivate auf Basis der in der schematisierten Formel I gezeigten Einheiten

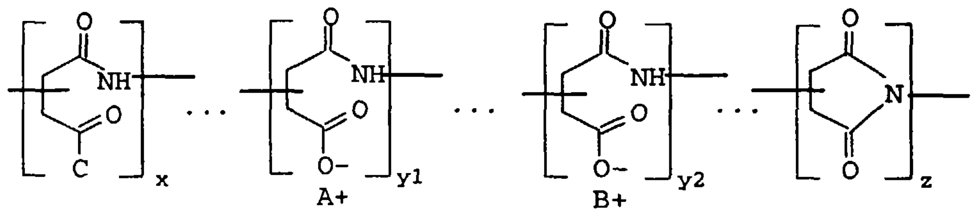 (I)

wobei die Reihenfolge der Einheiten beliebig ist,
die Summe x + y1 + y2 + z = 100 ist und

| | |
|---|---|
| x | für einen Wert von 30 bis 99,9 steht, |
| y1 | für einen Wert von 0,1 bis 50 steht, |
| y2 | für einen Wert von 5 bis 50 steht, |
| z | für einen Wert von 0 bis 30 steht, |
| A, B und C | die oben angegebenen Bedeutungen haben, |

oder deren Carbonsäure- und Polycarbonsäuresalze oder Quaternisierungsprodukte.

Die Herstellung der erfindungsgemäßen Polyasparaginsäurederivate erfolgt durch Umsetzung von Polyasparaginsäure oder von Polysuccinimid mit mindestens einem Amin vom Typ A und mindestens einem Amin vom Typ B in Wasser als Lösungsmittel. Wird für die Herstellung der erfindungsgemäßen Polyasparaginsäurederivate Polysuccinimid verwendet, so wird dies vorzugsweise durch Polykondensation von Asparaginsäure hergestellt, wobei hochmolekulares Polysuccinimid mit Molekulargewichten von bis zu 100 000 erhalten wird. Die Herstellung erfolgt nach bekannten Verfahren, z.B. durch Polykondensation in Gegenwart von Phosphorsäure, wie z.B. von P. Neri et al in J.Med.Chem.16, 893 (1973) beschrieben.

Zur Herstellung der Polyasparaginsäurederivate wird das Polysuccinimid oder die Polyasparaginsäure, z.B. bei einer Temperatur von 20 bis 70°C, in Wasser gegeben. Unter Rühren wird eine Aminmischung mit jeweils einem Amin der vorbeschriebenen Typen A und B zugegeben. Bei einer Temperatur von 30 bis 100°C, vorzugsweise 50 bis 80°C, lässt man, zweckmäßigerweise unter Stickstoffatmosphäre, reagieren, bis praktisch keine Imidstruktur mehr durch IR-Spektroskopie nachweisbar ist. Gegebenenfalls kann die Temperatur auch erhöht und/oder überschüssiges Amin entfernt werden (z.B. durch Destillation). Anschliessend kann das Produkt entweder mit einem Quaternisierungsmittel, wie z.B. Dimethylsulfat, quaternisiert oder mit einer Säure bzw. einer Polycarbonsäure, wie z.B. Milchsäure bzw. Polyasparaginsäure, neutralisiert werden. Das Produkt kann durch Sprühtrocknen oder durch Ausfällen mit einem organischen Lösungsmittel, wie Aceton, in fester Form erhalten werden.

Um Polymere mit guten Konditioniereigenschaften zu erhalten, müssen sowohl Amine vom Typ A, bevorzugt z.T. in Salzform, als auch vorzugsweise tertiäre Amine vom Typ B im Polymer enthalten sein, um gleichzeitig eine gute biologische Abbaubarkeit zu erreichen.

Gemäß einer vorgenannten, bevorzugten Ausführungsform verwendet man jedoch auch Polymere, die keine Ammoniumsalze aufweisen in der Kosmetik, vorzugsweise in der Haarkosmetik. Die Mengenverhältnisse von Amiden, Salzen und Imiden im Polymer können über die Reaktionsbedingungen gesteuert werden.

Die erfindungsgemäßen wasserdispergierbaren Verbindungen können in Form von wässrigen Mikrodispersionen mit Teilchendurchmessern von üblicherweise 5 bis 100 nm, insbesondere 10 bis 80 nm, und Feststoffgehalten von üblicherweise 0,1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Die Polyasparaginsäurederivate sind aufgrund ihrer ionischen Gruppierungen in der Regel leicht alkohol- und wasserlöslich oder zumindest ohne Zuhilfenahme von Emulgatoren in Alkohol und Wasser dispergierbar. Geladene kationische Gruppierungen lassen sich aus den vorliegenden tertiären Aminstickstoffatomen entweder durch Protonierung, z.B. mit Alkylierungsmitteln wie $C_1$-$C_4$-Alkylhalogeniden oder -sulfaten erzeugen. Beispiele solcher Alkylierungsmittel sind Ethylchlorid, Ethylbromid, Methylchlorid, Methylbromid, Dimethylsulfat und Diethylsulfat.

Des weiteren können die Asparaginsäurederivate auch, wie erwähnt, durch Umsetzung mit einer ein- oder mehrwertigen Carbonsäure, wie z.B. Milchsäure, Zitronensäure, Weinsäure etc., oder durch Umsetzung mit einer Polycarbonsäure, wie z.B. Polyasparaginsäure, Polyglutaminsäure, Carboxymethylcellulose, Polyacrylsäure etc., in leicht wasser- oder alkohollösliche oder dispergierbare Salze überführt werden.

Die erfindungsgemäßen Polyasparaginsäurederivate sind als Hilfsmittel in der Kosmetik und Pharmazie sowie als Beschichtungsmittel für die Textil-, Papier-, Druck- und Klebstoff-Industrie brauchbar. Sie sind insbesondere in der Haarkosmetik brauchbar und ziehen leicht auf das Haar auf. Zur Anwendung als Haarfestiger sind Polyasparaginsäurederivate bevorzugt, deren K-Wert (nach Fikentscher, Cellulose Chemie 13 (1932), S. 58-64) 20 bis 100 beträgt.

Gegenstand der vorliegenden Erfindung sind auch Haarbehandlungsmittel, die die erfindungsgemäßen Polyasparaginsäurederivate enthalten. Im allgeineinen enthält das Mittel die Polyasparaginsäurederivate in einer Menge von etwa 0,1 bis 30 Gew.-%, bezogen auf das Gesamtgewicht des Mittels.

Die erfindungsgemäßen Haarbehandlungsmittel liegen üblicherweise in Form einer wässrigen Dispersion oder in Form einer wässrigalkoholischen Lösung vor. Beispiele geeigneter Alkohole sind Ethanol, Propanol, Isopropanol etc. Die Mittel enthalten das Polyasparaginsäurederivat dann in einer Menge von etwa 0,1 bis 25 Gew.-%, bevorzugt 1 bis 15 Gew.-%.

Weiter enthalten die erfindungsgemäßen Haarbehandlungsmittel im allgemeinen übliche kosmetische Hilfsstoffe, beispielsweise Weichmacher, wie Glycerin und Glycol, Silikone, Emollienzien, Parfüms, UV-Absorber, Farbstoffe, Verdickungsmittel, antistatische Mittel, Mittel zur Verbesserung der Kämmbarkeit, Konservierungsmittel und Schaumstabilisatoren.

Gewünschtenfalls enthalten die Haarbehandlungsmittel zusätzlich mindestens ein herkömmliches Haarfestigerpolymer. Das auf das Gewicht bezogene Mischungsverhältnis von Polyasparaginsäurederivat zu dem anderen Haarfestigerpolymer beträgt dann insbesondere 1:0,1 bis 1:2. Das Haarbehandlungsmittel enthält die Polymermischung insbesondere in einer Menge von 0,1 bis 25 Gew.-%, vorzugsweise 1 bis 15 Gew.-%, enthält.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel wird so gering wie möglich gehalten, um den VOC-Gehalt (VOC = volatile organic compounds) nicht unnötig zu erhöhen. Sie beträgt im allgemeinen nicht mehr als 40 Gew.-%, bezogen auf das Gesamtgewicht des Mittels.

Die erfindungsgemäßen Polyasparaginsäurederivate und diese enthaltende Mittel besitzen den Vorteil, daß sie einerseits den Haaren die gewünschte Festigkeit verleihen und andererseits die Polymere leichter auswaschbar (redispergierbar) sind als die Polymere des Standes der Technik. Darüber hinaus lassen sich Haarbehandlungsmittel mit einem VOC-Gehalt von weniger als 60 Gew.-% und auch rein wässrige Formulierungen herstellen, selbst wenn sie als Haarspray formuliert sind.

Die Erfindung soll durch die nachfolgenden, nicht einschränkenden Beispiele erläutert werden:

## Beispiele

Der K-Wert wurde nach E. Fikentscher, Cellulose-Chemie 13 (1932), S. 58-64 an einer 1%igen NMP-Lösung mit einem pH-Wert von 7 und bei einer Temperatur von 25°C gemessen.

### a) Herstellungsbeispiele

Beispiel 1: (Methode A)

In einem Dreihalskolben mit Rührer, Tropftrichter und Rückflußkühler werden 97 g Polysuccinimid in 300 g NMP (N-Methylpyrrolidon) bei Raumtemperatur unter Stickstoffatmosphäre gelöst. Unter leichtem Erwärmen auf etwa 30 bis 40°C wird 85 g (1 mol) Piperidin bei einem pH-Wert von weniger als 9,2 langsam zugetropft. Das Reaktionsgemisch wird bei etwa 20 bis 40°C 16-20 Stunden gerührt. Anschließend wird NMP bei 120°C im Vakuum abdestilliert, so daß eine ca. 50%ige Lösung entsteht. Die NMP-Lösung wird unter Rühren langsam in 2000 ml Aceton getropft. Das ausgefallene Produkt wird abgesaugt, 3-mal mit Aceton gewaschen und bei 20 bis 40°C im Vakuum getrocknet. Man erhält ein weißes Pulver, das in Wasser sowie Wasser/Ethanol-Gemischen sehr gut löslich ist.

Die Beispiele 2 bis 4 der Tabelle 1 wurden analog hergestellt.

Beispiel 6: (Methode B)

In einem Dreihaiskolben mit Rührer, Tropftrichter und Rückflußkühler werden 97 g Polysuccinimid in 400 g Wasser vorgelegt. Bei 60 bis 70°C wird unter Stickstoffatmosphäre 109,1 g (1,1 mol) Cyclohexylamin bei einem pH-Wert von weniger als 9,2 langsam zugetropft. Anschließend wird das Reaktionsgemisch bei 80 bis 100°C solange gerührt, bis im IR-Spektrum praktisch keine Imid-Struktur mehr nachweisbar ist. Der Aminüberschuß wird zusammen mit Wasser bei

110°C abdestilliert. Es entsteht eine 35 bis 40%ige Polyasparaginsäurederivat-Lösung.

Die Beispiele 5, 7 und 8 der Tabelle 1 wurden analog hergestellt.

Tabelle 1

| Beispiel Nr. | Si. mol | Pi. mol | Mo. mol | N-MPi. mol | Cha. mol | t-BG. mol | Eda. mol | K-Wert (1% NMP) | Methode |
|---|---|---|---|---|---|---|---|---|---|
| 1 | 1 | 1,0 | - | - | - | - | - | 23,0 | A |
| 2 | 1 | 0,7 | - | - | 0,3 | - | - | 24,1 | A |
| 3 | 1 | - | 1,0 | - | - | - | - | 23,4 | A |
| 4 | 1 | - | 0,7 | 0,3 | - | - | 0,03 | 27,3 | A |
| 5 | 1 | - | 0,7 | 0,3 | - | - | 0,03 | 25,4 | B |
| 6 | 1 | - | - | - | 1,1 | - | - | 23,2 | B |
| 7 | 1 | - | 0,7 | - | - | 0,35 | - | 22,6 | B |
| 8 | 1 | - | 0,5 | - | 0,5 | - | 0,03 | 25,4 | B |

Si.: Succinimideinheiten im Polymer
Pi.: Piperidin
Mo.: Morpholin
N-MPi: N-Methylpiperazin
Cha.: Cyclohexylamin
t-BG.: tert.-Butylglycinat
Eda.: Ethylendiamin
NMP: N-Methylpyrrolidon

b) Anwendungsbeispiele

Um die Verwendung der erfindungsgemäßen Polyasparaginsäurederivate als Haarfestiger zu demonstrieren, wurden folgende Formulierungen hergestellt (VOC = volatile organic compounds):

| A) VOC 80 Aerosol-Haarspray: | |
|---|---|
| Polyasparaginsäurederivate 1-8 nach Tabelle 1 | 5 Gew.-% |
| Ethanol abs. | 50 Gew.-% |
| Wasser | 15 Gew.-% |
| Dimethylether | 30 Gew.-% |

| B) VOC 55 Aerosol-Haarspray: | |
|---|---|
| Polasparaginsäurederivate 1-8 nach Tabelle 1 | 5 Gew.-% |
| Ethanol abs. | 25 Gew-% |
| Wasser | 40 Gew.-% |
| Dimethylether | 30 Gew.-% |

| C) VOC 55 Pumpspray: | |
|---|---|
| Polyasparaginsäurederivate 1-8 nach Tabelle 1 | 5 Gew.-% |
| Ethanol abs. | 55 Gew.-% |
| Wasser | 40 Gew.-% |

Die folgenden anwendungstechnischen Eigenschaften wurden an der vorgenannten Formulierung A) als Beispiel untersucht.

- Curl-retention = Festigungswirkung an Haarsträhnen in Lockenform bei hoher Luftfeuchte (90%):
  Die Curl-Retention ist ein Maß für die Haarfestigungswirkung. Sie wird im Modellversuch gemessen an Haarlocken, erzeugt durch eine übliche Wasserwelle an ca. 15 cm langen Haaren, die mit Formulierung A) aus 10 cm Entfernung 4 sec lang besprüht werden. Nach 5-stündiger Behandlung der aufgehängten Locken in einer Klimakammer (25°C, 90% relative Luftfeuchte) wird die relative Verformung (Aufweitung) der Locken, bezogen auf die ursprüngliche Form, festgestellt. Ein hoher Wert bedeutet hohe Festigungswirkung, d.h. 100% entspricht einem Erhalt der ursprünglichen Form der aufgehängten Locke, 0% entspricht einem völlig gestreckten Haar.

- Biegefestigkeit
  Die Messung erfolgt mit 10 verschiedenen Haarsträhnen, die etwa gleich schwer (2 g) und gleich lang (24 cm) sind. Es handelt sich um mitteleuropäisches braunes Haar. Die Haarsträhnen werden 1 Stunde in eine Lösung aus 50% Ethanol und 50% Wasser gelegt, danach wird zweimal mit Texapon® NSO Lösung (der Fa. Henkel KgaA) (10% Feststoff) schamponiert und mit 40°C warmem Wasser ausgespült. Die nassen Haarsträhnen werden durchgekämmt und an der Luft bei Raumtemperatur getrocknet. Nach dem Trocknen werden die Haarsträhnen gewogen und in eine Lösung der Formulierung A) getaucht, wobei durch mehrmaliges Eintauchen und Herausnehmen eine gleichmäßige Verteilung sichergestellt ist.

Die überschüssige Lösung wird zwischen Daumen und Zeigefinger abgedrückt und anschließend zwischen Filterpapier sorgfältig abgedrückt, so daß eine Gewichtszunahme von 0,4 bis 0,5 g zu verzeichnen ist. Danach werden die Haarsträhnen so geformt, daß sie einen runden Querschnitt erhalten. Es erfolgt die Trocknung im Klimaschrank bei 20°C und 75% relativer Luftfeuchtigkeit. Nach 12 Stunden werden die Strähnen aus dem Klimaschrank genommen und sofort der Härtemessung unterzogen. Die Haarsträhne wird dazu auf 2-zylindrische Rollen (Durchmesser 6 mm) gelegt, die waagrecht zueinander im Abstand von 9 cm angebracht sind. Genau in der Mitte zwischen den beiden Auflagen wird nun von oben durch eine zylindrische Rolle (Durchmesser 6 cm) so lange mit stetig steigender Kraft auf die Haarsträhnen gedrückt, bis diese durchbrechen. Die zum Durchbrechen erforderliche Kraft wird gemessen. Nach dem Durchbrechen der Haarsträhne wird die Strähne entlastet, wodurch sich diese wieder streckt. Nun wird erneut stetig die Kraft erhöht, bis die Strähne zum zweiten Mal durchbricht.

Die Ergebnisse der anwendungstechnischen Untersuchung sind in Tabelle 2 wiedergegeben.

Tabelle 2

| Curl-Retention und Biegefestigkeit der Formulierung A) | | |
|---|---|---|
| Beispiel Nr. nach Tabelle 1 | Curl retention [%] | Biegefestigkeit [cN] |
| 1 | 63 | 107 |
| 2 | 68 | 103 |
| 3 | 71 | 115 |
| 4 | 45 | 118 |
| 5 | 39 | 82 |
| 6 | 52 | 100 |
| 7 | 38 | 98 |
| 8 | 55 | 109 |

Wie Tabelle 2 zeigt, weisen Haarfestigerformulierungen mit den erfindungsgemäßen Polyasparaginsäurederivaten eine gute Festigungswirkung auf.

**Patentansprüche**

1. Verwendung von wasserlöslichen und/oder wasserdispergierbaren Polyasparaginsäurederivaten auf Basis der in der schematisierten Formel I gezeigten Einheiten

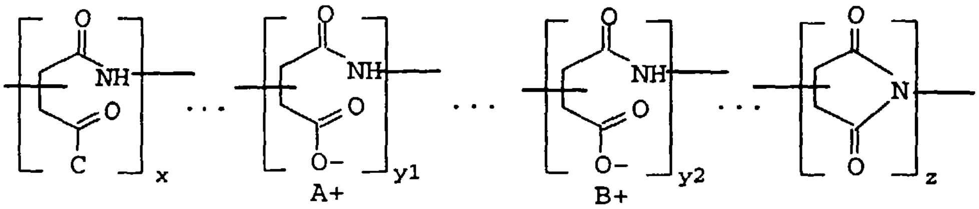

(I)

wobei
die Reihenfolge der Einheiten beliebig ist,
die Summe aus $x + y1 + y2 + z = 100$ ist und

| | |
|---|---|
| x | für einen Wert von 30 bis 100 steht, |
| y1 | für einen Wert von 0 bis 70 steht, |
| y2 | für einen Wert von 0 bis 50 steht, |
| z | für einen Wert von 0 bis 30 steht, |
| A | für mindestens ein primäres oder sekundäres nichtaromatisches Amin steht, |
| B | für mindestens ein Amin mit einer tertiären Aminogruppe steht, |
| C | für einen von einem Amin des Typs A durch Abspaltung eines Aminwasserstoffs abgeleiteten Rest steht, |

oder deren Carbonsäure- und Polycarbonsäuresalze oder Quaternisierungsprodukte,
in der Kosmetik, insbesondere in der Haarkosmetik.

2. Verwendung nach Anspruch 1, wobei

| | |
|---|---|
| x | für einen Wert von 50 bis 99,9 steht, |
| y1 | für einen Wert von 0,1 bis 50 steht, |
| y2 | für einen Wert von 5 bis 50 steht, |
| z | für einen Wert von 0 bis 15 steht. |

3. Verwendung nach Anspruch 1 oder 2, wobei das Amin A ausgewählt ist unter

1) primären Aminen der Formel II

$$R^1\text{-}NH_2 \qquad \text{(II)}$$

worin $R^1$ für

a) Alkyl steht, welches mit 1, 2 oder 3 Gruppen substituiert sein kann, die unabhängig voneinander ausgewählt sind unter Amino, Hydroxy, Alkoxycarbonyl, Alkylaminocarbonyl, Cycloalkyl, Cycloalkenyl, Aryl oder einem 5- bis 7-gliedrigen gesättigten, ungesättigten oder aromatischen Heterocyclus, der 1, 2 oder 3 N-, S- und/oder O-Heteroatome aufweist, wobei die cyclischen Reste zusätzlich 1, 2, oder 3 Substituenten, unabhängig ausgewählt unter Alkyl, Alkoxy, Hydroxy und Hydroxyalkyl, aufweisen können und/oder die Alkylgruppe durch 1, 2 oder 3 nicht benachbarte Sauerstoffatome unterbrochen sein kann;

b) Cycloalkyl oder Cycloalkenyl steht, wobei diese Reste mit 1, 2 oder 3 der folgenden Gruppen substituiert sein können, die unabhängig voneinander ausgewählt sind unter Alkyl, Alkoxy, Hydroxy, Hydroxyalkyl oder $-COR^2$, worin $R^2$ für Alkyl, Alkoxy oder $NR^3R^4$ steht, wobei $R^3$ und $R^4$ unabhängig voneinander für Wasserstoff und Alkyl stehen;

c) Bicycloalkyl oder Bicycloalkenyl steht, wobei diese Reste mit 1, 2 oder 3 der unter b) als Substituenten für Cycloalkyl und Cycloalkenyl angegebenen Gruppen substituiert sein können;

d) einen 5- bis 7-gliedrigen, gesättigten Heterocyclus steht, der 1, 2 oder 3 Heteroatome, ausgewählt unter O, N und S aufweist und der gegebenenfalls 1, 2 oder 3 Substituenten aufweisen kann, die unabhängig voneinander ausgewählt sind unter Alkyl, Alkoxy, Hydroxy und Hydroxyalkyl;

2) sekundären Aminen der Formel III

$$R^5 - NH - R^6 \qquad (III)$$

worin

$R^5$ und $R^6$ unabhängig voneinander die für $R^1$ angegebenen Bedeutungen annehmen können, oder
$R^5$ und $R^6$ zusammen mit dem Stickstoffatom, an das sie gebunden sind für einen 5- bis 7-gliedrigen, gesättigten oder ungesättigten Heterocyclus stehen, der 1, 2 oder 3 Heteroatome oder -gruppen, unabhängig voneinander ausgewählt unter $NR^7$, $NCOR^7$, O und S, aufweisen kann, wobei $R^7$ für Wasserstoff, Alkyl oder Hydroxyalkyl steht, und/oder wobei der Heterocyclus durch 1, 2 oder 3 Substituenten, unabhängig voneinander ausgewählt unter Alkyl, Alkoxy, Hydroxy und Hydroxyalkyl, substituiert sein kann.

**4.** Verwendung nach einem der Ansprüche 1 bis 3, wobei das Amin A ausgewählt ist unter:

1) primären Aminen der Formel II, worin $R^1$ für Alkyl oder Cycloalkyl steht, wobei die Alkylgruppe mit einer Alkoxycarbonylgruppe oder mit einer weiteren Aminogruppe substituiert sein kann;
2) sekundären Aminen der Formel III, worin $R^5$ und $R^6$ zusammen mit dem Stickstoffatom, an das sie gebunden sind für einen 5- bis 7-gliedrigen, gesättigten oder ungesättigten Heterocyclus stehen, der 1, 2 oder 3 Heteroatome oder -gruppen, unabhängig voneinander ausgewählt unter $NR^7$ und O aufweisen kann, wobei $R^7$ für Wasserstoff, Alkyl oder Hydroxyalkyl steht.

**5.** Verwendung nach einem der Ansprüche 1 bis 4, wobei das Amin B ausgewählt ist unter

1) tertiären Amin der Formel IV

$$NR^8R^9R^{10} \qquad (IV)$$

worin

$R^8$, $R^9$ und $R^{10}$ unabhängig voneinander die für $R^1$ angegebenen Bedeutungen annehmen können, oder
$R^8$ und $R^9$ zusammen mit dem Stickstoffatom, an das sie gebunden sind, für einen 5- bis 7-gliedrigen gesättigten oder ungesättigten Heterocyclus stehen, der 1, 2 oder 3 Heteroatome, ausgewählt unter $NR^{11}$, $NCOR^{11}$ und O, wobei $R^{11}$ für Wasserstoff oder Alkyl steht, aufweisen kann, und/oder der durch 1, 2 oder 3 Substituenten, unabhängig voneinander ausgewählt unter Alkyl, Alkoxy, Hydroxy und Hydroxyalkyl, substituiert sein kann,

2) Diaminen der Formel V

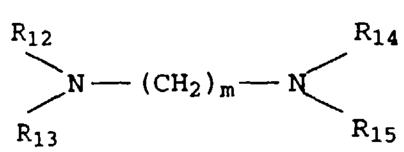

(V)

wobei

m für eine ganze Zahl von 2 bis 6 steht,

| | |
|---|---|
| $R^{12}$ und $R^{13}$ | unabhängig voneinander für Wasserstoff und Alkyl stehen, |
| $R^{14}$ und $R^{15}$ | unabhängig voneinander für Alkyl stehen oder $R^{14}$ und $R^{15}$ zusammen mit dem Stickstoffatom, an das sie gebunden sind, für einen 5- bis 7-gliedrigen gesättigten Heterocyclus stehen, der 1, 2 oder 3 Heteroatome, unabhängig ausgewählt unter N, O und S, aufweist, |

3) tertiären Fettaminen der Formel VI

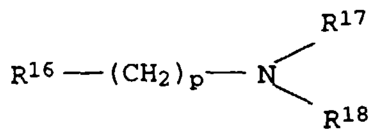

VI

worin

| | |
|---|---|
| p | für eine ganze Zahl von 6 bis 23 steht, |
| $R^{16}$ | für Wasserstoff oder den Kohlenwasserstoffrest einer gesättigten oder ungesättigten Fettsäure steht, |
| $R^{17}$ und $R^{18}$ | unabhängig voneinander für Alkyl stehen. |

**6.** Verwendung nach einem der Ansprüche 1 bis 5, wobei die Summe von y1 + y2 für einen Wert von 50 oder grösser, bevorzugt für einen Wert von 70 oder grösser, steht.

**7.** Verwendung nach einem der Ansprüche 1 bis 6, wobei der K-Wert der Polyasparaginsäurederivate 20 bis 100 beträgt.

**8.** Verwendung nach einem der Ansprüche 1 bis 7 als Haarfestiger.

**9.** Wasserlösliche und/oder wasserdispergierbare Polyasparaginsäurederivate auf Basis der in der schematisierten Formel I gezeigten Einheiten

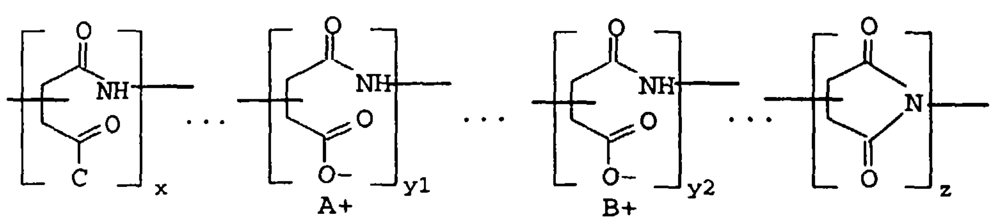

(I)

wobei die Reihenfolge der Einheiten beliebig ist,
die Summe x + y1 + y2 + z = 100 ist und

| | |
|---|---|
| x | für einen Wert von 30 bis 99,9 steht, |
| y1 | für einen Wert von 0,1 bis 50 steht, |
| y2 | für einen Wert von 5 bis 50 steht, |
| z | für einen Wert von 0 bis 30 steht, |
| A, B und C | die in Anspruch 1 und/oder einem der Ansprüche 3 bis 5 angegebenen Bedeutungen haben, |

oder deren Carbonsäure- und Polycarbonsäuresalze oder Quaternisierungsprodukte.

**10.** Verfahren zur Herstellung der Polyasparaginsäurederivate nach Anspruch 9, dadurch gekennzeichnet, daß man Polyasparaginsäure oder Polysuccinimid mit mindestens einem Amin vom Typ A und mindestens einem Amin vom Typ B zur Reaktion bringt.

**11.** Verfahren nach Anspruch 10, dadurch gekennzeichnet, daß das erhaltene Polyasparaginsäurederivat mit einem Quaternisierungsmittel umgesetzt wird.

**12.** Verfahren nach Anspruch 10, dadurch gekennzeichnet, daß das erhaltene Polyasparaginsäurederivat mit einer Carbonsäure oder Polycarbonsäure umgesetzt wird.

**13.** Haarbehandlungsmittel, das wenigstens ein Polyasparaginsäurederivat nach Anspruch 9 enthält.

**14.** Haarbehandlungsmittel nach Anspruch 13, das das/die Polyasparaginsäurederivat(e) in einer Menge von 0,1 bis 25 Gew.-%, vorzugsweise 1 bis 15 Gew.-%, enthält.

**15.** Haarbehandlungsmittel nach einem der Ansprüche 13 oder 14, das weitere übliche kosmetische Hilfsstoffe, ausgewählt unter Weichmachern, Silikonen, Emollienzien, Parfüms, UV-Absorbern, Farbstoffen, Verdickungsmitteln, Antistatika, Mitteln zur Verbesserung der Kämmbarkeit, Konservierungsmitteln und Schaumstabilisatoren enthält.

**16.** Haarbehandlungsmittel nach einem der Ansprüche 13 bis 15, das zusätzlich mindestens ein anderes Haarfestigerpolymer enthält.

**17.** Haarbehandlungsmittel nach einem der Ansprüche 13 bis 16, das in Form einer wässrigen Dispersion oder einer wässrig-alkoholischen Lösung vorliegt.

**18.** Haarbehandlungsmittel nach Anspruch 17 in Form eines Haarsprays.

Europäisches Patentamt

# EUROPÄISCHER RECHERCHENBERICHT

Nummer der Anmeldung
EP 97 11 3350

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,X | EP 0 767 191 A (MITSUI TOATSU CHEMICALS)<br>* das ganze Dokument *<br>--- | 1-18 | A61K7/06<br>C08G69/10<br>C08G73/06 |
| D,A | DE 36 12 102 A (CESKOSLOVENSKA AKADEMIE VED)<br>* Ansprüche *<br>--- | 9-12 | |
| D,A | EP 0 406 623 A (ROEHM GMBH)<br>* Seite 4, Zeile 20 - Seite 5, Zeile 33 *<br>--- | 9-12 | |
| D,A | DATABASE WPI<br>Section Ch, Week 9440<br>Derwent Publications Ltd., London, GB;<br>Class A23, AN 94-322255<br>XP002045386<br>& JP 06 248 072 A (AJINOMOTO KK) ,<br>6.September 1994<br>* Zusammenfassung *<br>--- | 1,9-12 | |
| A | US 3 846 380 A (Y. FUJIMOTO ET AL.)<br>* Spalte 3, Zeile 65 - Spalte 4, Zeile 37 *<br>--- | 9-12 | **RECHERCHIERTE SACHGEBIETE (Int.Cl.6)** |
| A | GB 2 005 705 A (L'OREAL)<br>* Ansprüche *<br>--- | 1-18 | A61K<br>C08G |
| A | FR 2 424 292 A (L'OREAL)<br>* Ansprüche *<br>--- | 1-18 | |
| A | US 4 735 797 A (J. GROLLIER ET AL.)<br>--- | 1-18 | |
| A | E. W. NEUSE ET AL.: "Water-soluble polyamides as potential drug carriers"<br>DIE ANGEWANDTE MAKROMOLEKULARE CHEMIE, Bd. 192, Nr. 3300, 1991, BASEL,<br>Seiten 35-50, XP002045582<br>* das ganze Dokument *<br>----- | 1-18 | |

Der vorliegende Recherchenbericht wurde für alle Patentansprüche erstellt

| Recherchenort | Abschlußdatum der Recherche | Prüfer |
|---|---|---|
| BERLIN | 6.November 1997 | Boeker, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)